(19) Europäisches Patentamt
European Patent Office
Office européen des brevets

(11) **EP 1 229 017 B1**

(12) **EUROPÄISCHE PATENTSCHRIFT**

(45) Veröffentlichungstag und Bekanntmachung des Hinweises auf die Patenterteilung:
**12.05.2004 Patentblatt 2004/20**

(51) Int Cl.⁷: **C07C 211/65**, C07F 3/00

(21) Anmeldenummer: **02000991.6**

(22) Anmeldetag: **17.01.2002**

(54) **Erdalkalimetall-alkylendiamide, Verfahren zu deren Herstellung und deren Verwendung**

Alkylendiamides of alkaline-earth-metals, process for their preparation and use

Derivés d'alkylendiamide de métaux alcalins-terreux, procédé de préparation et utilisation

(84) Benannte Vertragsstaaten:
**AT BE CH CY DE DK ES FI FR GB GR IE IT LI LU MC NL PT SE TR**

(30) Priorität: **01.02.2001 DE 10104807**

(43) Veröffentlichungstag der Anmeldung:
**07.08.2002 Patentblatt 2002/32**

(73) Patentinhaber: **Chemetall GmbH**
**60487 Frankfurt am Main (DE)**

(72) Erfinder:
- **Weiss, Wilfried, Dr.**
  **61440 Oberursel (DE)**
- **Dawidowski, Dirk**
  **60439 Frankfurt (DE)**

(74) Vertreter: **Uppena, Franz, Dr. et al**
**Dynamit Nobel Aktiengesellschaft,**
**Patente, Marken & Lizenzen**
**53839 Troisdorf (DE)**

(56) Entgegenhaltungen:
**US-A- 3 992 561** **US-A- 4 020 115**

EP 1 229 017 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft Erdalkalimetall-alkylendiamide, ein Verfahren zu deren Herstellung und deren Verwendung.

Die Erdalkaliamide sind starke Metallierungsmittel und dienen u.a. als Ausgangsstoffe für die Herstellung Erdalkalimetallalkoxiden (M.F. Lappert et al., "Metall and Metalloid Amides", John Wiley & Sons, NY 1980).

Die Alkoxide (oder Alkoholate) der Erdalkalimetalle finden z.B. Verwendung als Precurser zur Herstellung dünner Metalloxidschichten (nach dem CVD oder dem Sol-Gel Verfahren) für Elektronikmaterialien, insbesondere der Hochtemperatur-Supraleiter (W.A. Hermann, *Angew. Chem.* ***1995***, 107, 2371).

Erdalkalialkoxide werden auch als Additive bei der anionischen Polymerisation von Dienen mit Butyllithium eingesetzt; hier bewirkt der Zusatz von Erdalkalialkoxiden einen hohen trans-Anteil des Polymerisates, was sich vorteilhaft auf die Eigenschaften der Endprodukte, z.B. Reifen, auswirkt (US 5100965, US 4020115 und US 3992561).

Daneben sind die Erdalkalialkoxide aufgrund ihrer basischen Eigenschaften und oft guten Löslichkeiten in Ethem und Kohlenwasserstoffen auch als Reagenzien in Deprotonierungs- und Alkoxilierungsreaktionen sowie Kondensationen vielseitig einsetzbar (D.C. Bradley, R.C. Mehrotra, D.P. Gaur, *Metal Alkoxides, Academic Press, NY,* ***1978***; US 4555498).

Bisher bekannte Synthesemethoden für z.B. Bariumalkoxide gehen aus von:

- den Metallen:

$$Ba + 2\ RO\text{-}H \Rightarrow Ba(OR)_2 + H_2 \qquad (1)$$

- den Metallamiden (Amidroute):

$$Ba(NH_2)_2 + 2\ RO\text{-}H \Rightarrow Ba(OR)_2 + 2\ NH_3 \qquad (2)$$

- den Metalljodiden (Salzmetathese):

$$BaJ_2 + 2\ RO\text{-}K \Rightarrow Ba(OR)_2 + 2\ KJ \qquad (3)$$

Die Reaktion (1) ist sehr langsam und führt zu "Komplexen" $Ba(OR)_2$*$(ROH)_n$, die bei hohen Temperaturen zersetzt werden müssen.

Bei der Reaktion (2) wird das Metall zunächst in flüssigen Ammoniak (T < - 33 °C) zum Amid gelöst und dann in Folge zum Alkoxid umgesetzt, was die Nachteile einer Tieftemperaturreaktion beinhaltet.

Bei der Reaktion (3) schließlich ist die Jodid-Herstellung unwirtschaftlich.

Aufgabe der Erfindung ist es, die Nachteile des Standes der Technik zu überwinden und Stoffe bereitzustellen, die in einem relativ einfachen Verfahren die Herstellung von Erdalkalimetallalkoxiden ermöglichen.

Gelöst wird die Aufgabe durch Erdalkalimetall-alkylendiamide, wobei als Erdalkalimetalle Ba, Sr und Ca eingesetzt werden können. Erdalkalimetall-alkylendiamide können mit einem Alkohol ohne weiteres zum entsprechenden Erdalkalimetallalkoxid umgesetzt werden. Bevorzugte Erdalkalimetall-alkylendiamide leiten sich von den primären Alkylendiaminen ab. Besonders bevorzugt sind Erdalkalimetall-ethylendiamide.

Überraschend wurde gefunden, dass sich die Erdalkalimetalle Ba, Sr und Ca sehr einfach in Ethylendiamin lösen lassen, wobei sich das entsprechende Erdalkalimetall-ethylendiamid und Wasserstoff bilden:

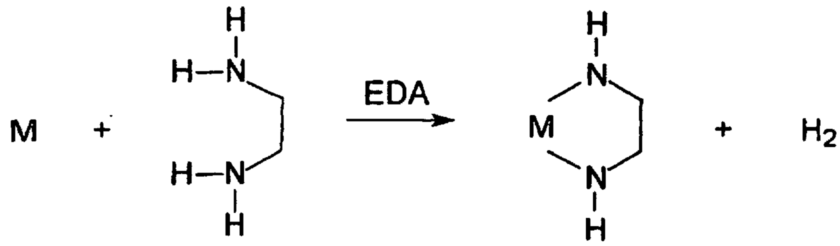

M = Ba, Sr, Ca
EDA = Ethylendiamin

Bevorzugt wird das Ethylendiamin vorgelegt und das Erdalkalimetall in Form von z. B. Pulver, Granulat oder Stücken zudosiert. Bevorzugt wird die Reaktion unter Inertgasatmosphäre durchgeführt. Bevorzugt wird das Ethylendiamin zum Erdalkalimetall im Überschuss bei einem molaren Verhältnis von 2 : 1 bis 50 : 1 eingesetzt. Eine bevorzugte Reaktionstemperatur liegt zwischen 8,5 °C und 118 °C, also in dem Bereich, in dem Ethylendiamin flüssig ist. Prinzipiell lässt sich die Reaktion auch bei tieferen Temperaturen mit festem Ethylendiamin als Festkörperreaktion oder bei höheren Temperaturen mit gasförmigem Ethylendiamin durchführen. Besonders bevorzugt ist der Temperaturbereich von 10 bis 40 °C; die Reaktion kann also vorteilhaft bei Raumtemperatur durchgeführt werden. Ein Maß für die Reaktionsgeschwindigkeit ist die Wasserstoffentwicklung. Die Reaktionsgeschwindigkeit der exothermen Reaktion kann durch die Zudosierung des Erdalkalimetalls und in begrenztem Umfang durch Kühlung des Reaktionsgefäßes gesteuert werden. Das Erdalkalimetall-ethylendiamid fällt als unlöslicher grauer Feststoff an, der durch Filtration und/oder Eindampfung und Trocknung isoliert werden kann.

Die Reaktion lässt sich am besten mit Barium als Erdalkalimetall und Ethylendiamin als Alkylendiamin durchführen.

Prinzipiell kann die Umsetzung auch mit anderen Alkylendiaminen als Ethylendiamin durchgeführt werden, allerdings sind hier die Reaktionsgeschwindigkeiten langsamer; so benötigt man z.B. für die Auflösung von Barium in Propylendiamin (zum Barium-propylendiamid) am Siedepunkt unter Normaldruck mehrere Tage. Eine Reaktionsbeschleunigung lässt sich durch Erhöhung der Temperatur bei gleichzeitiger Druckerhöhung erreichen.

Eine Verwendung der erfindungsgemäßen Erdalkalimetall-alkylendiamide ist deren Einsatz in der Synthese von Erdalkalimetallalkoxiden.

Dazu werden die Erdalkalimetall-alkylendiamide (hier: ein Erdalkalimetall-ethylendiamid) mit einem Alkohol zum entsprechenden Erdalkalimetallalkoxid umgesetzt.

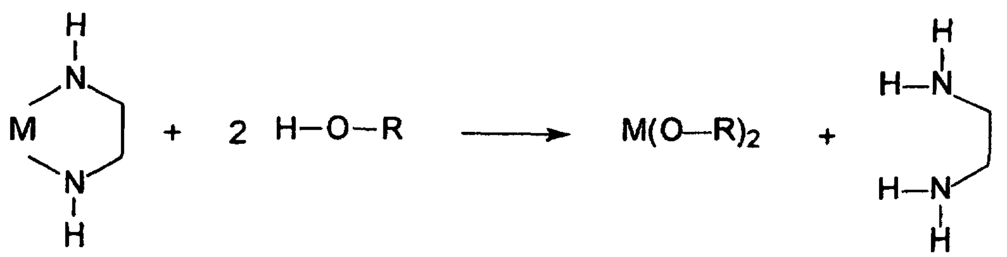

Bevorzugt ist dabei eine stöchiometrische Menge Alkohol, nichtstöchiometrische Alkoholmengen führen neben dem gewünschten Produkt zu Komplexen. Die Umsetzung mit Alkohol kann unverdünnt, in verbliebenem, überschüssigem Alkylendiamin oder in einem Lösungsmittel erfolgen. Als Lösungsmittel können aliphatische Kohlenwasserstoffe (cyclische oder acyclische) mit 5 bis 12 C-Atomen oder aromatische Kohlenwasserstoffe mit 6 bis 12 C-Atomen und/oder Ether eingesetzt werden.

Als Kohlenwasserstoffe können z.B. eine oder mehrere der Verbindungen Pentan, Cyclopentan, Hexan, Heptan, Octan, Cyclohexan, Toluol, Xylol, Cumol, Ethylbenzol oder Tetralin eingesetzt werden.

Als Ether können z.B. eine oder mehrere der Verbindungen Tetrahydrofuran (THF), 2-Methyl-THF, Tetrahydropyran, Diethylether, Diisopropylether, Dibutylether, Dioxan, Methyl-tert-butylether, Glycolether (wie Monoglyme, Diglyme) oder Gemische davon eingesetzt werden.

Eine Isolierung des Erdalkalialkoxides kann durch Eindampfung und Trocknung erfolgen, wobei das Alkylendiamin recycliert werden kann. Das Erdalkalialkoxid kann entweder als Feststoff bereitgestellt werden oder kann in einem Lösungsmittel gelöst und als fertige Lösung bereitgestellt werden.

Die Erfindung wird im folgenden anhand von Beispielen näher erläutert.

**Beispiel 1: Herstellung von Barium-ethylendiamid**

In einem 1-1-Reaktionskolben mit Magnetrührer wurden bei Raumtemperatur unter Argon 700 g (11,65 mol) Ethylendiamin (EDA) vorgelegt. Dazu wurden innerhalb von 5 Stunden 80 g (0,58 mol) Barium-Späne portionsweise zugefügt. Bei der exothermen Reaktion färbte sich die Lösung zunächst blau, schlug dann um in grau, und das entstehende Barium-ethylen-diamid (Ba-EDA) fiel aus. Der entstehende Wasserstoff (0,58 mol) war ein Maß für die Reaktionsgeschwindigkeit und zeigte das Reaktionsende an. Die erhaltene Reaktionssuspension wurde bei 100 °C im Ölpumpenvakuum eingedampft. Es verblieben 120 g Ba-EDA mit einem Gehalt von 95 %.

**Beispiel 2: Herstellung von Barium-ethylendiamid**

Analog Beispiel 1 wurde eine Ba-EDA-Suspension hergestellt, die über eine G-3 Fritte filtriert wurde. Der Rückstand wurde im Ölpumpenvakuum bei Raumtemperatur getrocknet. Das erhaltene Ba-EDA (126 g) hatte einen Gehalt von 90 %.

**Beispiel 3: Herstellung von Strontium-ethylendiamid**

In einem 500-ml-Reaktionskolben mit Magnetrührer wurden bei Raumtemperatur unter Argon 220 g EDA (3,66 mol) mit 16 g (0,18 mol) Strontium-Stücke versetzt und bis zum Erliegen der Wasserstoffentwicklung (0,18 mol) mehrere Tage gerührt. Die erhaltene graue Reaktionssuspension wurde bei 120 °C im Ölpumpenvakuum eingedampft. Es verblieben 29 g Sr-EDA als 70 %-iges Pulver.

**Beispiel 4: Herstellung von Calcium-ethylendiamid**

In einem 500-ml-Reaktionskolben mit Magnetrührer wurden bei Raumtemperatur unter Argon 220 g EDA (3,66 mol) mit 7 g (0,18 mol) Calcium-Granulat versetzt und bis zum Erliegen der Wasserstoffentwicklung 10 Tage bei RT gerührt. Innerhalb dieser Zeit hatte sich 0,1 mol des Calciums zu einem hellgrauen Pulver (Calcium-ethylendiamid) umgesetzt.

**Beispiel 5: Herstellung von Barium-propylendiamid**

In einem 250-ml-Reaktionskolben mit Magnetrührer wurden bei Raumtemperatur unter Argon 58 g 1,3-Propylendiamin (0,78 mol) mit 1,8 g (0,013 mol) Barium-Granulat versetzt. Die Wasserstoffentwicklung setzte langsam ein und die Lösung trübte sich durch ausfallendes Barium-propylendiamid. Innerhalb von 4 Tagen gingen 3,5 mmol Barium in Lösung, was einer Ausbeute von 25 % entspricht.

**Beispiel 6: Herstellung von Barium-tert-butoxid**

12 g Ba-EDA aus Beispiel 1 (95 %-ig, d.h. 58 mmol Ba-EDA) wurden in 175 g Cyclohexan suspendiert und innerhalb von 15 Minuten mit einem Gemisch aus 8,7 g tert-Butanol (116 mmol) und 8,7 g Cyclohexan versetzt und zur vollständigen Umsetzung 1 Stunde zum Sieden erhitzt. Nach Einengen bei 120 °C im Ölpumpenvakuum verblieben 17 g Barium-tert-butoxid als hellbraunes Pulver. Dieses wurde in 3 Portionen geteilt, die jeweils aus Hexan, Cyclohexan und Toluol umkristallisiert wurden. Die Löslichkeiten wurden wie folgt festgestellt:

| | |
|---|---|
| Löslichkeit in Hexan | 5,9 % |
| Löslichkeit in Cyclohexan | 11,3 % |
| Löslichkeit in Toluol | 11,6 % |

**Beispiel 7: Herstellung von Barium-tert-amoxid**

44 g Ba-EDA aus Beispiel 1 (95 %-ig, d.h. 213 mmol Ba-EDA) wurden in 1500 ml Cyclohexan supendiert und bei Raumtemperatur innerhalb von 90 Minuten mit 38 g (430 mmol) tert-Amylalkohol versetzt und anschließend 1 h refluxiert. Nach Einengen bei 120 °C in Ölpumpenvakuum verblieben 62 g Barium-tert-amoxid als hellbraunes Pulver. Dieses wurde in 3 Portionen geteilt, die jeweils aus Hexan, Cyclohexan und Toluol umkristallisiert wurden. Die Löslichkeiten wurden wie folgt festgestellt:

| | |
|---|---|
| Löslichkeit in Hexan | 3,7 % |
| Löslichkeit in Cyclohexan | 4,0 % |
| Löslichkeit in Toluol | 3,4 % |

**Beispiel 8: Herstellung von Barium-2-ethylhexanolat**

In einem 1-l-Schlenkkolben wurden 72 g Ba-EDA aus Beispiel 1 (365 mmol) in 570 g Cyclohexan suspendiert und innerhalb von 3 Stunden mit 92,7 g 2-Ethylhexanol (712 mmol) versetzt. Der Reaktionsansatz erwärmte sich dabei leicht von 25 auf 30 °C und das Barium-2-etylhexanolat ging in Lösung. Der Reaktionsansatz wurde bei 120 °C eingeengt und im Ölpumpenvakuum getrocknet. Das zurückbleibende zähe Öl wurde in 400 g Cyclohexan gelöst und

vom überschüssigen Ba-EDA abfiltriert. Erhalten wurden 510 g Lösung mit einem Gehalt von 325 mmol Barium-2-ethylhexanolat, was einer Ausbeute von 91 % entspricht.

Die Löslichkeiten wurden wie folgt festgestellt:

| | |
|---|---|
| Löslichkeit in Hexan | 17,8 % |
| Löslichkeit in Cyclohexan | 49,5 % |
| Löslichkeit in Toluol | 16,6 % |

## Patentansprüche

1. Erdalkalimetall-alkylendiamide, mit Erdalkalimetall = Ba, Sr, Ca.

2. Erdalkalimetall-ethylendiamide, mit Erdalkalimetall = Ba, Sr, Ca.

3. Bariumethylendiamid.

4. Verfahren zur Herstellung von Erdalkalimetall-alkylendiamiden, **dadurch gekennzeichnet, dass** ein Erdalkalimetall, ausgewählt aus Ba, Sr oder Ca in einem Alkylendiamin gelöst wird, wobei sich das entsprechende Erdalkalimetall-alkylendiamid bildet.

5. Verfahren nach Anspruch 4, **dadurch gekennzeichnet, dass** das Alkylendiamin zum Erdalkalimetall im Überschuss bei einem molaren Verhältnis von 2 : 1 bis 50 1 eingesetzt wird.

6. Verfahren nach Anspruch 4 oder 5, **dadurch gekennzeichnet, dass** die Reaktion bei einer Temperatur bis 200 °C und einem Druck bis 10 bar durchgeführt wird.

7. Verwendung von Erdalkalimetall-alkylendiamiden zur Herstellung von Erdalkalimetallalkoxiden.

8. Verfahren zur Herstellung von Erdalkalimetallalkoxiden, **dadurch gekennzeichnet, dass** ein Erdalkalimetall in einem Alkylendiamin gelöst wird, wobei sich das entsprechende Erdalkalimetall-alkylendiamid bildet, und das entstandene Erdalkalimetall-alkylendiamid mit einem Alkohol zum Erdalkalimetallalkoxid umgesetzt wird.

9. Verfahren nach Anspruch 8, **dadurch gekennzeichnet, dass** die Reaktion in einem Lösungsmittel durchgeführt wird.

10. Verfahren nach Anspruch 9, **dadurch gekennzeichnet, dass** als Lösungsmittel Kohlenwasserstoffe und/oder Ether eingesetzt werden.

11. Verfahren nach Anspruch 10, **dadurch gekennzeichnet, dass** als Kohlenwasserstoffe aliphatische Kohlenwasserstoffe (cyclische oder acyclische) mit 5 bis 12 C-Atomen oder aromatische Kohlenwasserstoffe mit 6 bis 12 C-Atomen eingesetzt werden.

12. Verfahren nach Anspruch 11, **dadurch gekennzeichnet, dass** als Kohlenwasserstoffe eine oder mehrere der Verbindungen Pentan, Cyclopentan, Hexan, Heptan, Octan, Cyclohexan, Toluol, Xylol, Cumol, Ethylbenzol oder Tetralin eingesetzt werden.

13. Verfahren nach Anspruch 10, **dadurch gekennzeichnet, dass** als Ether eine oder mehrere der Verbindungen Tetrahydrofuran (THF), 2-Methyl-THF, Tetrahydropyran, Diethylether, Diisopropylether, Dibutylether, Dioxan, Methyl-tert-butylether oder Glycolether eingesetzt werden.

## Claims

1. Alkaline-earth metal alkylene diamides, where alkaline-earth metal = Ba, Sr, Ca.

2. Alkaline-earth metal ethylene diamides, where alkaline-earth metal = Ba, Sr, Ca.

**3.** Barium ethylene diamide.

**4.** Process for the production of alkaline-earth metal alkylene diamides, **characterised in that** an alkaline-earth metal selected from Ba, Sr or Ca is dissolved in an alkylene diamine, the corresponding alkaline-earth metal alkylene diamide being formed.

**5.** Process according to claim 4, **characterised in that** the alkylene diamine is used in excess relative to the alkaline-earth metal, in a molar ratio of 2:1 to 50:1.

**6.** Process according to claim 4 or 5, **characterised in that** the reaction is performed at a temperature of up to 200 °C and under a pressure of up to 10 bar.

**7.** Use of alkaline-earth metal alkylene diamides for the production of alkaline-earth metal alkoxides.

**8.** Process for the production of alkaline-earth metal alkoxides, **characterised in that** an alkaline-earth metal is dissolved in an alkylene diamine, the corresponding alkaline-earth metal alkylene diamide being formed, and the resulting alkaline-earth metal alkylene diamide is transferred to the alkaline-earth metal alkoxide with an alcohol.

**9.** Process according to claim 8, **characterised in that** the reaction is performed in a solvent.

**10.** Process according to claim 9, **characterised in that** hydrocarbons and/or ethers are used as solvent.

**11.** Process according to claim 10, **characterised in that** aliphatic hydrocarbons (cyclic or acyclic) having 5 to 12 C atoms or aromatic hydrocarbons having 6 to 12 C atoms are used as hydrocarbons.

**12.** Process according to claim 11, **characterised in that** one or more of the compounds pentane, cyclopentane, hexane, heptane, octane, cyclohexane, toluene, xylene, cumene, ethyl benzene or tetralin are used as hydrocarbons.

**13.** Process according to claim 10, **characterised in that** one or more of the compounds tetrahydrofuran (THF), 2-methyl-THF, tetrahydropyran, diethyl ether, diisopropyl ether, dibutyl ether, dioxan, methyl tert-butyl ether or glycol ether are used as ethers.

## Revendications

**1.** Alkylène-diamidure de métal alcalino-terreux, dans lequel le métal alcalino-terreux est du baryum, du strontium ou du calcium.

**2.** Ethylène-diamidure de métal alcalino-terreux, dans lequel le métal alcalino-terreux est du baryum, du strontium ou du calcium.

**3.** Ethylène-diamidure de baryum.

**4.** Procédé de préparation d'alkylène-diamidures de métal alcalino-terreux, **caractérisé en ce qu'**on dissout dans une alkylène-diamine un métal alcalino-terreux choisi parmi du baryum, du strontium et du calcium, ce par quoi il se forme l'alkylène-diamidure de métal alcalino-terreux correspondant.

**5.** Procédé conforme à la revendication 4, **caractérisé en ce qu'**on emploie l'alkylène-diamine en excès par rapport au métal alcalino-terreux, le rapport molaire de celle-là à celui-ci valant de 2/1 à 50/1.

**6.** Procédé conforme à la revendication 4 ou 5, **caractérisé en ce qu'**on effectue la réaction à une température qui peut valoir jusqu'à 200 °C et sous une pression qui peut valoir jusqu'à 10 bars.

**7.** Emploi d'alkylène-diamidures de métal alcalino-terreux pour la préparation d'alcoolates de métal alcalino-terreux.

**8.** Procédé de préparation d'alcoolates de métal alcalino-terreux, **caractérisé en ce qu'**on dissout dans une alkylène-diamine un métal alcalino-terreux, ce par quoi il se forme l'alkylène-diamidure de métal alcalino-terreux correspondant, et l'on fait réagir avec un alcool l'alkylène-diamidure de métal alcalino-terreux ainsi formé, pour obtenir un alcoolate de métal alcalino-terreux.

**9.** Procédé conforme à la revendication 8, **caractérisé en ce qu'**on effectue la réaction dans un solvant.

**10.** Procédé conforme à la revendication 9, **caractérisé en ce que** le solvant est choisi parmi les hydrocarbures et/ou les éthers.

**11.** Procédé conforme à la revendication 10, **caractérisé en ce que** les hydrocarbures sont des hydrocarbures aliphatiques, cycliques ou acycliques, en $C_{5-12}$ ou des hydrocarbures aromatiques en $C_{6-12}$.

**12.** Procédé conforme à la revendication 11, **caractérisé en ce qu'**on emploie, à titre d'hydrocarbures, un ou plusieurs des composés suivants : pentane, cyclopentane, hexane, heptane, octane, cyclohexane, toluène, xylène, cumène, éthylbenzène, tétraline.

**13.** Procédé conforme à la revendication 10, **caractérisé en ce qu'**on emploie, à titre d'éthers, un ou plusieurs des composés suivants : tétrahydrofurane (THF), 2-méthyl-THF, tétrahydropyrane, diéthyl-éther, diisopropyléther, dibutyl-éther, dioxane, méthyl-tertiobutyl-éther, éther de glycol.